# EUROPEAN PATENT SPECIFICATION

(11) **EP 2 692 922 B1**
(45) Date of publication and mention of the grant of the patent: **04.04.2018**
(21) Application number: 12178692.5
(22) Date of filing: 31.07.2012
(51) Int. Cl.: A61F 13/513, A61F 13/534, A61F 13/536, D04H 1/495, D04H 1/492, A61F 13/47, A61F 13/511

(54) **Channelled nonwoven with reduced surface expansion of liquid for the production of sanitary towels and relative process of manufacture**
Mit Kanälen versehener Vliesstoff mit reduzierter Oberflächenausdehnung von Flüssigkeit zur Herstellung von Sanitärhandtüchern und entsprechendes Herstellungsverfahren
Matériau canaliculé non tissé avec expansion réduite de surface de liquide pour la production de serviettes hygiéniques et son procédé de fabrication

(43) Date of publication of application: 05.02.2014
(73) Proprietor: Fa-Ma Jersey S.p.A., 59100 Prato (IT)
(72) Inventor: Maranghi, Marco, 59100 Prato (IT)
(74) Representative: Petruzziello, Aldo

(56) References cited:
- DE-A1- 10 361 339
- GB-A- 2 114 054
- US-A1- 2007 226 970
- US-A1- 2007 298 220

## Description

The present invention relates to sanitary towels for physiological liquids, with high absorbency, provided with a reduced surface expansion of the liquid absorbed through capillarity thanks to the particular manufacture of the surface layer (topsheet) in cotton or similar absorbent materials in natural fibres.

Sanitary towels in cotton are more breathable with respect to conventional sanitary towels made with synthetic materials, and therefore generally more comfortable. Moreover these sanitary towels in cotton are suitable for all those who exhibit dermatological problems in contact with synthetic materials.

The sanitary towels in cotton currently present on the market are formed by a thin porous surface layer, in perforated and non-perforated nonwoven cotton, also known as topsheet, which has a function of covering with the sole purpose of forming a hypoallergenic contact layer. Below this topsheet an intermediate absorbent/filtering layer in cotton or in airlaid of cellulose fibres, with greater thickness, is positioned, which forms the core thereof, and a base polymeric film as support, also having the function of providing a surface on which to apply the adhesive strips for the attachment of said sanitary towel to the panty.

These sanitary towels can also be provided with so-called "wings", also adhesive, which ensure an even better attachment of the sanitary towel to the panty. These wings are formed by the topsheet layer supported by the aforementioned polymeric film without any intermediate absorbent layer.

To date this topsheet is obtained from cotton fibres, in the form of a pad, which is subsequently subjected to hydroentangling or water needling (spunlace), thus forming a layer of cotton where the fibres are bound one to the other.

The spunlace technology binds the fibres using jets of water exiting from a jet strip of nozzles at high pressure, in general 60-80 bars, appropriately distanced, for example by 0.8 mm, with a diameter of approximately 0.08 mm. Said jets of water pass through the fibrous pad, binding one to the other the fibres below the high-pressure jets, and the nonwoven obtained in this way is resistant and at the same time soft, with grams per square metre varying generally from 20 to 60 g/m².

Since cotton is notoriously an absorbent material with high capillarity, this type of sanitary towel suffers the disadvantage of exhibiting a high expansion of the liquid which traverses it with the consequent disadvantage of having a widening of the stain of liquid as far as the wings, soiling them.

The object of the present invention is to provide a sanitary towel able to overcome, at least in part, the disadvantages suffered by the prior art, in particular a sanitary towel in cotton having a reduced surface expansion of the liquid absorbed, in particular on the wings, ensuring at the same time a high absorbency of liquid.

A further object is that of providing such a sanitary towel which can be made with a simple and economical process, easy to perform.

These objects are achieved by the sanitary towel with wings in absorbent material of natural fibres such as cotton or viscose, with microperforations or plain, which has the features of the annexed independent claim 1.

Advantageous embodiments of the invention are disclosed by the dependent claims. The object of the present invention is a sanitary towel with wings comprising as topsheet a layer of absorbent nonwoven material in natural fibres such as cotton and/or any other natural fibre and mixtures thereof with viscose or polylactic acid or another fibre suitable for the purpose, constituted by a hydroentangled pad, said topsheet having a density which varies transversely and is provided with a first plurality of longitudinal lines in the form of grooves along which said textile has a higher density of fibres with respect to the rest of the textile, each one of said lines of said first plurality of lines being alternated with a respective longitudinal line of a second plurality of lines along which said textile has a lower density of fibres.

The higher density of fibres along the first plurality of lines is due to the fact that the fibres there are more compressed and the textile has a smaller thickness: this means that the capillarity due to the greater closeness between fibre and fibre tends to increase in the longitudinal direction creating lines or bands of channelling for the liquid.

The lower density of fibres along each line of said second plurality of lines is due to the fact that the fibres there are less compressed and more distanced one from the other: this means that the transfer of liquid from one fibrella (fibre) to the other is more difficult, slowing down the flow of the capillarity in the transverse direction.

In practice the longitudinal lines with lower density represent lines of barrier to the flow of the liquid in a transverse direction (perpendicular to these lines) coming from the adjacent lines with higher density, facilitating therefore the channelling of the liquid along the lines with higher density (and high absorbency).

The specific alternation of lines with different density of fibres is obtained by means of a specific process of preparation of the nonwoven textile which forms the topsheet which will now be described.

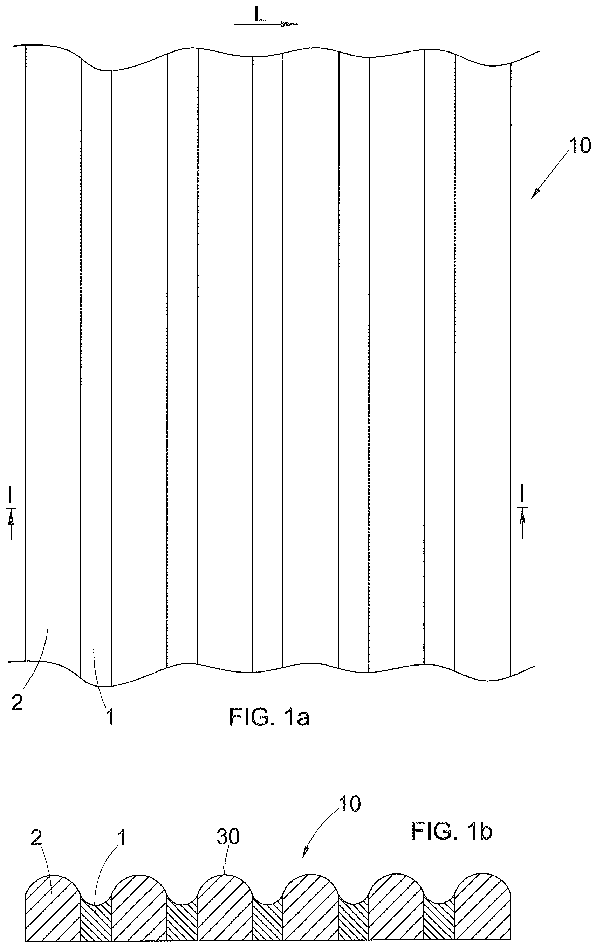

This process comprises:
- a first step (a) of hydroentangling with water of a layer of fibres with jets of water adjacent one to the other, suitable for applying pressures lower than or equal to 60 bars to said layer, in order to obtain a light cohesion between the fibres, and
- a second step (b) of hydroentangling with water of the hydroentangled pad obtained from the first step (a), using jets of water with pressures higher than or equal to 100 bars or with higher flow rate with respect to step (a), appropriately distanced one from the other, with a pitch (along the transverse line L of the topsheet of Fig. 1a) of at least 0.5 mm one from the other, preferably of at least 1 mm, more preferably of at least 2 mm.

The jets of water at high pressure or with higher flow rate of step (b) mean that the innermost fibres of the topsheet layer are also cohered, which does not occur in the first step where the pressures are low and the binding is superficial.

The jets of water at high pressure or with higher flow rate of step (b) are generally more distanced one from the other and with a greater nozzle diameter with respect to the jets of water with low pressure or with lower flow rate of step (a): in this way the jets of water at high pressure once again go to hit only a part of the zones previously hit by the jets at lower pressure, therefore creating a textile with more compressed zones (of high density) alternated with less compressed zones (of lower density).

Steps (a) and (b) are applied on the same side 30 of the topsheet (Fig. 1b). Said steps can be performed in a single phase using at the same time jets of water both with high and low pressure by means of the simultaneous use of different nozzles, with different diameter, as will subsequently be described in detail.

The layer of fibres to be subjected to step (a) can be pre-needled, needle-punched in the form of a pad or also only be constituted by a plurality of folded or carded webs.

The treatment with jets of water used in steps (a) and (b) of the present process is a technology known in the art and also referred to as water binding (also referred to as spunlacing, hydroentangling, etc.). Refer for example to US 3,485,706 or what is described in the patent application EP 1359241 incorporated here in full for reference.

In the art entangling is generally performed at pressures generally around 60-80 bars, using nozzles of small diameter, for example 0.08 mm, placed almost in contact one with the other, therefore with a different arrangement with respect to step b).

Reference will now be made to the accompanying drawings which are purely an example, and therefore non-limiting, of embodiments of the present invention, in which:
Fig. 1a is an enlarged view from above, and partially interrupted transversely, of a topsheet in accordance with the present invention;
Fig. 1b is a sectioned view, enlarged, of the topsheet of Fig. 1a taken along line I-I;
Figs. 2a-2b are schematic perspective views of the unit of hydroentangling in two different phases of preparation of the topsheet of the present invention;
Figs. 3a-3b are views from above of the topsheet of the invention respectively at the moment of formation of the stain of liquid and in a subsequent moment of expansion of said stain;
Figs. 4 a)-e) are views from above of a conventional topsheet a), in microperforated 35 g/m² cotton and of some topsheets b)-e) of the invention, in microperforated 35 g/m² cotton, after a predetermined time from the addition of test liquid;
Fig. 5 is a view from above, enlarged and partially interrupted, of an arrangement of low-pressure nozzles in the respective nozzle-holder jet strip;
Fig. 6 is a view from above, enlarged and partially interrupted, of an arrangement of high-pressure nozzles in the respective nozzle-holder jet strip;
Figs. 7 a)-b) is a view from above, enlarged and partially interrupted, of two different arrangements of high-pressure nozzles in the respective nozzle-holder jet strip;
Fig. 8 is a view from above, enlarged and partially interrupted, of a single arrangement of high-pressure and low-pressure nozzles in a single nozzle-holder jet strip.

As illustrated in Figs. 1a and 1b, the topsheet layer 10 in accordance with the present invention is a layer provided with a first plurality of longitudinal lines 1, along which the fibres are more compressed (higher density of fibres and smaller thickness), and of a second plurality of longitudinal lines 2, alternated with the longitudinal lines 1, along which the fibres are less compressed (lower density of fibres and greater thickness).

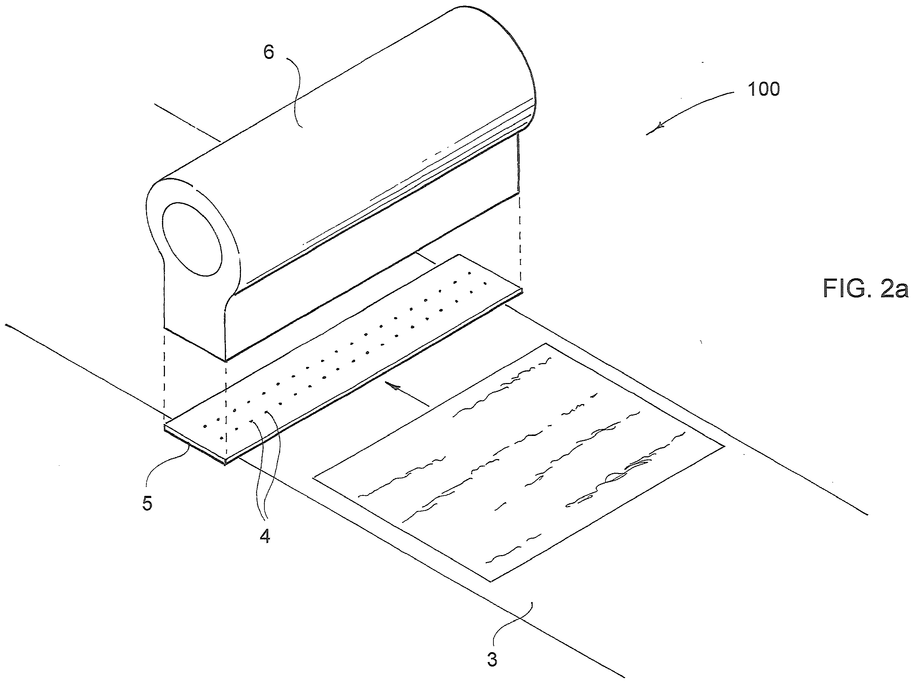

Referring to Figs. 2a-2b, the topsheet 10 in nonwoven cotton is prepared according to the following method: the starting layer which will go to form the topsheet is a layer of fibres coming generally from a carding plant, also known as "web", which is placed on a conveyor belt 3 which supplies the hydroentangling section 100.

In said section 100 the fibres of said layer (web) are subjected to a first phase of entangling with water by means of pressurised jets which allow the fibres to cohere one with the other: this is necessary in that in the web coming from the carding plant the fibres are only joined through reciprocal adhesion but break and separate if subjected to traction.

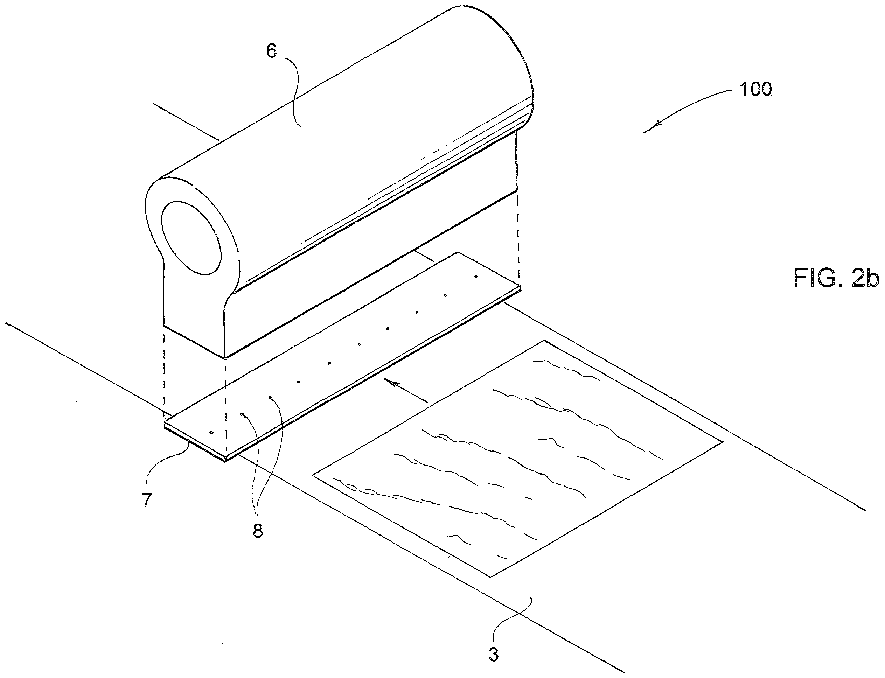

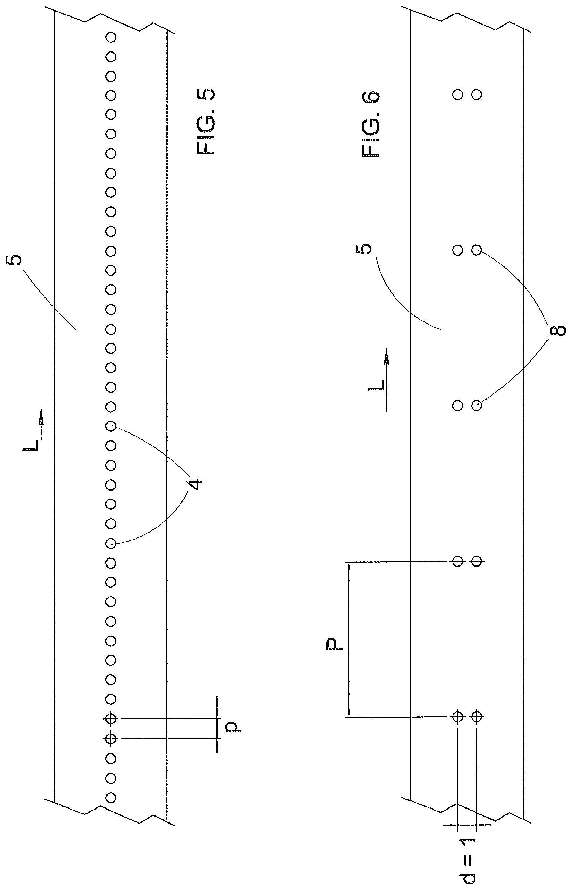

Referring to Figs. 2b) and 5, in this first phase of hydroentangling a plurality of nozzles 4 with diameter (Φ) of approximately 0.08 mm and a distance between nozzle and nozzle (pitch, p) (along the transverse line L in Fig. 5) equal to approximately 0.8 mm are used. These nozzles 4 are holes formed in a steel plate 5 which acts as jet strip, and can be arranged in a single row (Fig. 5) or in several rows, for example two (Fig. 2a) appropriately distanced one from the other.

Other examples of diameter (Φ) of nozzle 4 are 0.06 mm and 0.10 mm.

In one embodiment said rectangular jet strip 5 generally has a width of 20-25 mm, thickness of approximately 0.5 mm and length of approximately 2.5 m, and is perforated to a design with holes of different pitch and diameter according to the type of binding which is to be performed and of average grams per square metre value which is required for the topsheet 10.
The abovementioned dimensions of the jet strip 5 are not binding for the purpose of the present invention and can also vary by a few centimetres up to even 3/6 metres according to the size of the type of machine on which it is to be mounted.

Said jet strip 5 is mounted on a nozzle-holder injector 6 which acts as distributor of the flow of water, which is placed, in fixed position, above the conveyor belt 3 which makes the material advance in the direction of the arrow indicated in Fig. 2a. Said distributor 6 goes to supply with water the plate 5 wherein the holes of the nozzles 4 have been formed.

In this first step a) water is used at a pressure which is not high, generally between 30 and 40 bars, up to even approximately 60 bars.

The entangled layer of fibres obtained in said first step (a) is then passed to a zone where part of the water absorbed can be discharged, and subsequently sent again into the entangling unit where the first jet strip 5 has been replaced with a second jet strip 7 (Figs. 2b, 6, 7a), 7b)) suitable for supporting nozzles 8 with greater diameter with respect to the previous nozzles 4 and distanced one from the other with a greater pitch (P), (along the transverse line L in Fig. 6).

By way of an example the nozzles 8 for high pressure can have a diameter (Φ) of 0.8 mm or of 0.12 mm, 0.14 mm, 0.16 mm, and can be distanced with a pitch (P) which varies from 1 mm up to 5 mm, preferably 2-3 mm. Said nozzles 8 can also be arranged in several horizontal rows.

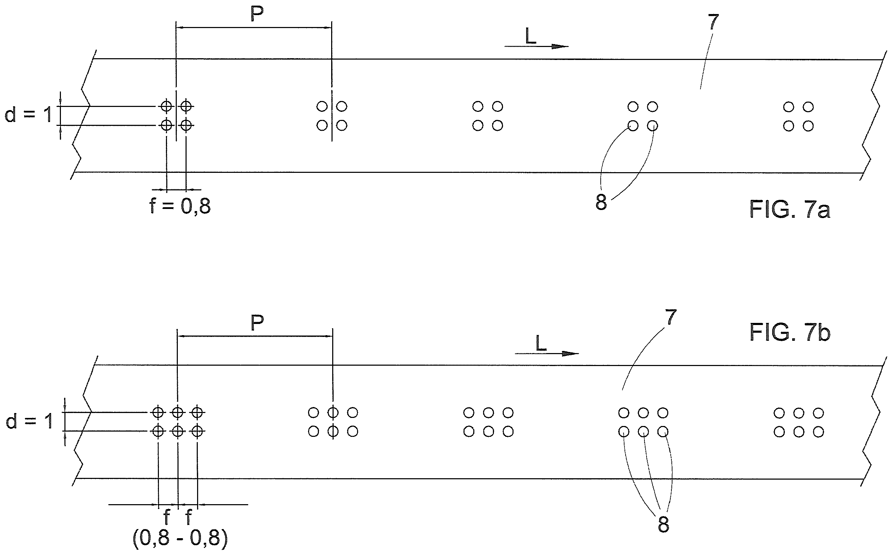

Figures 6, 7a), 7b) illustrate different arrangements of high-pressure nozzles 8 in the same number of nozzle-holder jet strips 7.

Fig. 6 shows a first embodiment of the jet strip 7 containing nozzles 8 with diameter of 0.12 mm, which are arranged at a distance P of 2 or 3 mm one from the other. The nozzles 8 are also arranged in two horizontal rows (along the line L), which have a distance (d), one from the other, of approximately 1 mm.

Fig. 7a shows a second embodiment of the jet strip 7 containing nozzles 8 with diameter of 0.12 mm, arranged in two rows which have a distance (d), one from the other, of approximately 1 mm.

Next to each nozzle 8 of each row a second nozzle 8 is positioned, at a distance (f) for example of approximately 0.8 mm. In this way groups of nozzles 8 are formed along the line L, each one formed by four nozzles 8 practically adjacent one to the other. Each group is distant one from the other by a pitch (P) which can be equal to 2, 3, 5 mm.

Fig. 7b shows a third embodiment of the jet strip 7 containing nozzles 8 with diameter of 0.12 mm, arranged in two rows which have a distance (d), one from the other, of approximately 1 mm.

Next to each nozzle 8 of each row two other nozzles 8 are positioned, at a distance (f) for example of approximately 0.8 mm. In this way groups of nozzles 8 are formed, each one formed by six nozzles 8. Each group is distant one from the other by a pitch (P), along the line L, which can be equal to 2, 3, 5 mm.

In the embodiments of Figs. 6, 7a), 7b) the distance f between the high-pressure nozzles 8, positioned one alongside the other, can also be equal to 0.6 mm rather than 0.8 mm.

The abovementioned dimensions of the jet strip 7 are not binding for the purpose of the present invention and can also vary by a few centimetres up to even 3/6 metres according to the size of the type of machine on which it is to be mounted.

Both jet strips 5 and 7 are positioned in a transverse direction to the movement of the conveyor belt 3, as shown in Figs. 2a and 2b with the arrow, in such a way that, when the belt 3 moves, the jets of water exiting from the nozzles 4 and 8 hit the entire pad of fibres along longitudinal lines. The dimensions of these jet strips 5 and 7 can be for example 2 m in length and 25 cm in width.

In practice the hydroentangling at higher pressures, generally higher than 100 bars, or with higher flow rates with respect to the first entangling (a), takes place only in specific points or zones (longitudinal lines 1) of the topsheet thanks to the arrangement of the nozzles 8 in the jet strip 7: for this reason the density in said longitudinal lines 1 is higher with respect to the zones (longitudinal lines 2) not hit by the high-pressure jets.

Through the nozzle-holder device 6 and the alternation of nozzles 4 and 8 which operate at different pressures, different pressures are applied along the longitudinal lines of the nozzles: said lines represent lines (or rows) of sealing (bonding) along which the surface fibres bind more or less with the underlying fibres according to the pressure used.

Subsequently the topsheet obtained from said second step (b) is air-dried and wound on a reel.

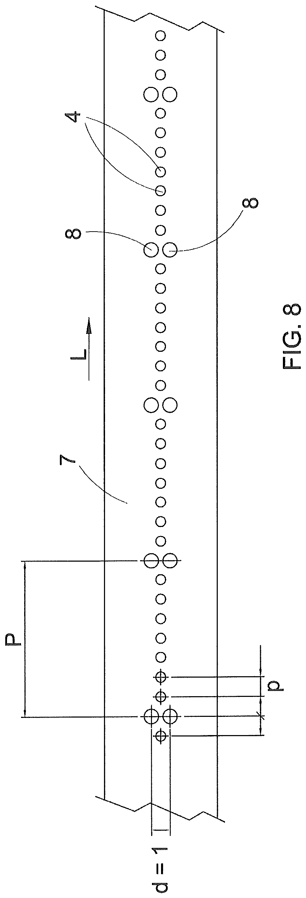

As mentioned above, it is also possible for said steps (a) and (b) to be performed at the same time in a single phase using jets of water which hit the pad with different flow rates and therefore with different hydraulic powers, by means of a single jet strip 7 where both types of nozzles 4 and 8 are arranged, as illustrated in Fig. 8.

In fact, considering a jet strip 7 of the type of Fig. 8 with holes of different diameter, for example 0.08 mm 0.12 mm, with the same pressure a greater hydraulic energy of approximately double the power will be obtained for the holes with larger diameter, in that the expression of the power on the diameter of the holes is squared.

For example with 1000 holes with diameter of 0.08 mm at the pressure of 100 bars a flow rate of 361 1/h is obtained; the same 1000 holes with diameter of 0.12 mm at the same pressure of 100 bars will have a flow rate of 811 1/h: an increase of 125% of hydraulic energy.

In practice, using the same pressure yet a single jet strip with two different types of holes, the same effect obtained at the end of step b) is achieved.

In said embodiment referred to Fig. 8, the nozzles for low pressure 4 have a diameter (Φ) of approximately 0.08 mm and a distance between nozzle and nozzle (p) equal to approximately 0.8 mm and are only placed along a single row, while the nozzles 8 of diameter of 0.12 mm and are placed in two rows which have a distance (d), one from the other, of approximately 1 mm, each nozzle 8 being placed at a pitch P one from the other, along the line L, which can be equal to 2, 3, 5 mm.

In practice, using the same pressure yet utilising simultaneously nozzles 4 at low pressure/flow rate and nozzles 8 at high pressure/flow rate we have
- greater binding effect with the holes 8 of larger diameter for greater hydraulic power of the flow of water due to a greater passage: a greater and more extended barrier effect with a greater longitudinal capillarity due to the greater vicinity of the large holes 8;
- lower binding effect with the holes 4 of smaller diameter, in the drawing with diameter 0.08: the holes 4 with smaller diameter have a lower flow rate of the water and therefore a lower hydroentangling energy.

Nevertheless the embodiments of jet strip 7 illustrated in Figs. 7a and 7b are preferred, where at each pitch P there is a group of nozzles 8 placed very close one to the other.

In fact, using a jet strip 7 where the larger holes 8 are arranged in several horizontal rows and form groups, and where each group is distanced from the other by a pitch P, there will be a greater density of holes per cm with respect to the configuration in a single row.

In this configuration with groups the distance of the holes 8, inside the group, is an important process parameter: in fact the holes 8 with pitch 0.8 mm will have a density of holes of 12.5 holes per cm, while the holes 8 with pitch 1.0 mm will have a density of 10 holes per cm.

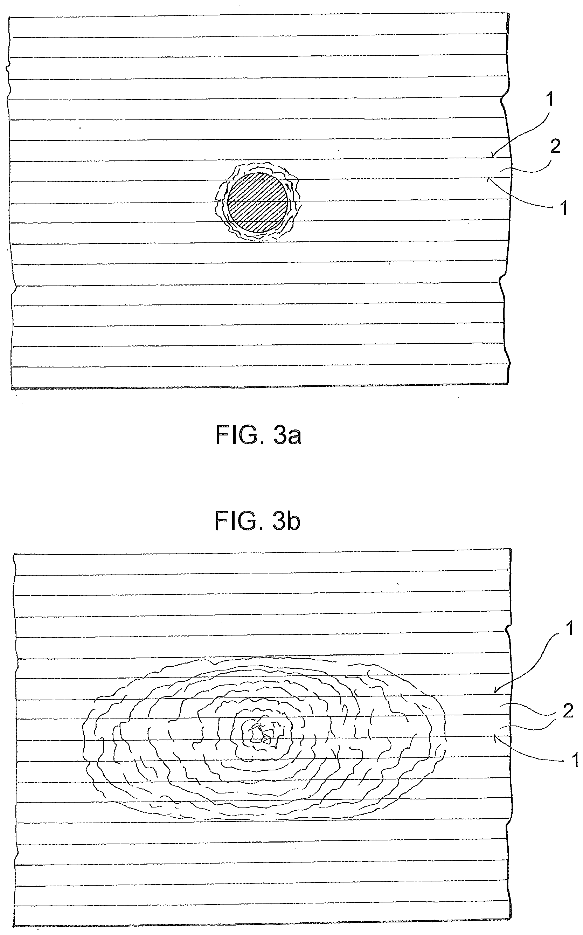

As illustrated in Figs. 3a and 3b, while the lines with lower density 2 represent longitudinal channellings for the liquid along which it can expand, the lines with greater density 1 constitute a barrier to the passage of the liquid in transverse direction with respect to said longitudinal lines 2.

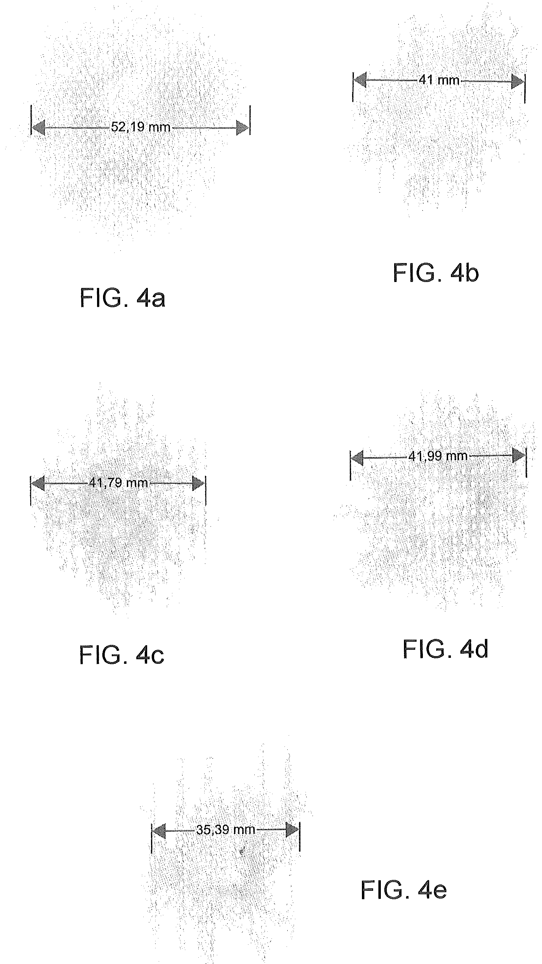

The Applicant has surprisingly found by means of tests that the transverse expansion of liquid is considerably reduced when a natural nonwoven absorbent material such as cotton is subjected to the process of the present invention.
In fact, as shown in Figures 4 a)-e), the transverse diffusion of the stain is reduced a great deal by changing from a conventional topsheet (Fig. 4a)) to topsheets made according to the invention (Figs. 4 b)-e)).

Moreover the topsheet of Figs. 4 b)-d) in accordance with the invention show a diffusion of liquid substantially similar one to the other: said topsheets were obtained by using the jet strip 7 of Fig. 6 respectively with pitch P=3 mm, P=3 mm and P=2 mm.

The topsheet of Fig. 4e) in accordance with the invention, which shows the smaller transverse diffusion of liquid, was obtained by using the jet strip 7 of Fig. 7 with a pitch (P) equal to 5 mm.

Without wishing to be bound to any theory, it can be presumed that the wider the areas with higher density and binding 1 (made with several nozzles 8 adjacent one to the other), the greater the orientation of the capillarity in a longitudinal rather than a transverse direction. This prevents the fluid from diffusing through transverse capillarity to the sides of the topsheet, and therefore towards the wings of the sanitary towel, with considerable advantages.

This is illustrated schematically in Figs. 4 a)-e) which show the stains on various topsheet samples after a predetermined time from the addition of an appropriate test liquid which simulates the behaviour of blood: in Figure 4a) the conventional topsheet, in microperforated 35 g/m² cotton, hydroentangled at a single pressure of 60-80 bars with nozzles of the same diameter, has a stain with width much greater with respect to some topsheets b)-e) of the invention, in microperforated 35 g/m² cotton.

The cotton fibres used for the formation of the topsheet can be of any micronaire, for example 2 to 5, or can be a mixture of fibres with different micronaire.

Similarly to the above, in the case of a natural fibre different from cotton or in the case of mixtures of fibres of cotton, viscose, polylactic acid (PLA) or other similar fibres, the fibres used can be of any dimensions.

With the present process it is possible to obtain a topsheet with a conventional grams per square metre value where the weight is constant over the entire surface yet the density and the thickness are different according to whether in a zone of high density along the longitudinal lines 1 or in a zone with low density along the longitudinal lines 2.

In practice these zones along the longitudinal lines 1 create a transverse barrier effect and diffuse the fluid longitudinally: the greater the density differential between the lines 1 and the lines 2, the greater the channelling of the flows longitudinally.

Therefore the greater or smaller difference in density (g/cm³) between said first plurality of longitudinal lines 1 and said second plurality of lines 2 is to be chosen on the basis of the end use of the topsheet.

In general, in order to obtain good channelling, the difference in density is at least 5%, preferably at least 10%, more preferably around 15-20%, even if these values are not binding for the purpose of the present invention.

Using the nozzles 8 described in Fig. 7a with a pitch P of 3 mm for each group of nozzles, a topsheet sample was obtained, provided with a density differential equal to approximately 15% which exhibited good longitudinal channelling of the flow.

More particularly the density obtained on 35 g of cotton using nozzles 4 as in Fig. 5 with pressure at 60 bars, and nozzles 8 as in Fig. 7a with pressure at 100 bars, a density of 0.095 g/cm³ was obtained in the zones with high density (longitudinal lines 1) shown in Fig. 1a, and a density of 0.082 g/cm³ in the zones with low density (longitudinal lines 2), resulting in a density differential equal to 16% approximately.

In practice the Applicant, by means of tests, has noted that the transverse diffusion of the liquid appears to be further reduced when, in addition to a greater differentiation of density, there is also
- a greatest width possible of the zones with low density and the zones with high density, and/or
- a greater number of zones with high and low density in a transverse direction.

In fact the Applicant has observed that
- the greater the difference of density between the zones with high and low density, the greater the channelling of the fluids in the longitudinal direction;
- the greater the width of the zones (lines) with low and high density, the greater the barrier effect which is obtained;
- the greater the number of the zones (lines) with low and high density per linear centimetre in transverse direction, the greater the barrier effect.

### By resuming,

- **the first plurality of longitudinal lines 1 having a higher density of fibres is obtained at the end of step b) and the second plurality of longitudinal lines 2 having a lower density of fibres is obtained at the end of step a);**
- **the first plurality of longitudinal lines 1 having a higher density of fibres obtained at the end of step b) has a smaller thickness with respect to the thickness of the second plurality of longitudinal lines 2 obtained at the end of step a).**

Numerous detail variations and changes can be made to the present embodiment of the invention, within the reach of a person skilled in the art and in any case coming within the scope of the invention expressed by the annexed claims.

## Claims

1. Process for preparing a topsheet layer (10) in absorbent nonwoven material of natural fibres for a sanitary towel with wings for physiological liquids,
said topsheet layer (10) having a density which varies in the transverse direction and provided with a first plurality of longitudinal lines (1) in the form of grooves having a higher density of fibres and a smaller thickness with respect to that of a second plurality of longitudinal lines (2), each one alternated with each longitudinal line (1),
said process comprising
(a) a first hydroentangling of a layer of fibres with pressures lower than or equal to 60 bars, with jets of water adjacent one to the other, in order to obtain a light cohesion between the fibres resulting in a lightly hydroentangled pad,
(b) a second hydroentangling of the lightly hydroentangled pad obtained from step (a), using pressures higher than or equal to 100 bars and/or with jets of water with higher flow rate, said jets being distanced one from the other with a pitch of at least 0.5 mm.

2. Process according to claim 1 wherein the layer of fibres to be used in step (a) is pre-needled, needle-punched in the form of a pad or constituted by a plurality of folded or carded webs.

3. Process according to claim 1 or 2 wherein the layer of fibres to be used in step (a) is fed on a conveyor belt (3) coming from a carding unit.

4. Process according to any one of the preceding claims 1-3 wherein the nozzles (4) for the water jets used in step (a) have a diameter (Φ) of approximately 0.08 mm, are mounted on a first rectangular jet strip (5) placed over the conveyor belt (3) and are distanced one from the other by a distance (pitch, d) equal to approximately 0.8 mm.

5. Process according to any one of the preceding claims 1-4 wherein the nozzles (8) to be used in step (b) have a diameter comprised between 0.8 mm and 0.12 mm (Φ), are mounted on a second jet strip (7) and are distanced one from the other by a pitch (P) comprised between 1 mm and 5 mm, each of said nozzles (8) also being possibly adjacent to one or more nozzles (8) at each pitch (P).

6. Process according to claim 5 wherein the nozzles (8) in the jet strip (7) are placed in several horizontal rows at a distance (d) one from the other, each nozzle (8) of each row being adjacent to one or more nozzles (8) at a distance (f) one from the other so as to form groups of nozzles (8), each group being distant from the other by a pitch (P) which varies from 1 mm to 5 mm.

7. Process according to any one of the preceding claims wherein the first plurality of longitudinal lines (1) having a higher density of fibres is obtained at the end of step (b) and the second plurality of longitudinal lines (2) having a lower density of fibres is obtained at the end of step (a).

8. Process according to claim 7 wherein the first plurality of longitudinal lines (1) having a higher density of fibres obtained at the end of step b) has a smaller thickness with respect to the thickness of the second plurality of longitudinal lines (2) obtained at the end of step (a).

9. Sanitary towel with wings for physiological liquids comprising as topsheet layer (10) a layer of absorbent nonwoven material of natural fibres constituted by a pad, said topsheet having a density which varies in the transverse direction so as to obtain a reduced transverse capillarity
said material is provided with a first plurality of longitudinal lines (1), in the form of grooves, which are intercalated with a second plurality of longitudinal lines (2), each one of said longitudinal lines (2) being alternated with each longitudinal line (1),
**characterised in that**
said pad constituting the top sheet is an hydroentangled one,
and **in that**
the longitudinal groove lines (1) of said first plurality of longitudinal lines have a higher density of fibres with respect to said longitudinal lines (2) of said second plurality of lines,
said first plurality of longitudinal groove lines (1) of higher density of fibres have a smaller thickness with respect to the thickness of said second plurality of longitudinal lines (2),
said topsheet forming also said wings.

10. Sanitary towel according to claim 9 wherein the difference in density (g/cm³) between said first plurality of longitudinal lines (1) and said second plurality of lines (2) is at least 10%, preferably around 15-20%.

11. Sanitary towel according to any one of the preceding claims 9-10 wherein the natural fibres of the absorbent material of the topsheet (10) are cotton and/or any other natural fibre and their mixtures with viscose, polylactic acid (PLA) or other similar fibres.

12. Sanitary towel according to claim 9 or 10 or 11 wherein the micronaire of the cotton fibres of the absorbent material used for the formation of the topsheet (10) is comprised between 2 and 5, or is a mixture of fibres with different micronaire.

13. Sanitary towel according to any one of the preceding claims 9-12 **characterised in that** it has wings which are opposite in said transverse direction.

14. Topsheet (10) for sanitary towels for physiological liquids as defined in any one of the preceding claims 9-13.

## Patentansprüche

1. Verfahren zur Herstellung einer Oberblattschicht (10) in einem absorptionsfähigen Vliesstoffmaterial aus Naturfasern für eine Damenbinde mit Flügeln für physiologische Flüssigkeiten,
wobei die Oberblattschicht (10) eine Dichte aufweist, die in der Querrichtung variiert, und mit einer ersten Mehrzahl von Längslinien (1) in Form von Vertiefungen ausgestattet ist, die eine höhere Faserdichte und eine geringere Dicke im Vergleich mit derjenigen einer zweiten Mehrzahl von Längslinien (2) aufweisen, wobei jede mit jeder Längslinie (1) abwechselt,
wobei das Verfahren folgendes umfasst
(a) ein erstes Wasserstrahlverschlingen einer Schicht von Fasern mit Drucken, die geringer als oder gleich 60 bar sind, mit Strahlen von Wasser, die einander benachbart sind, um eine leichte Kohäsion zwischen den Fasern zu erhalten, was zu einer leicht wasserstrahlverschlungenen Matte führt,
(b) ein zweites Wasserstrahlverschlingen der leicht wasserstrahlverschlungenen Matte, die aus Schritt (a) erhalten worden ist, unter Anwendung von Drucken führt, die höher als oder gleich 100 bar sind und/oder mit Strahlen von Wasser mit einer höheren Strömungsrate, wobei die Strahlen einer voneinander mit einem Abstand von mindestens 0,5 mm beabstandet sind.

2. Verfahren nach Anspruch 1, wobei die Schicht Fasern, die in Schritt (a) verwendet werden soll, in Form einer Matte vorvernadelt, vernadelt oder durch eine Mehrzahl gefalteter oder kardierter Bahnen konstituiert ist.

3. Verfahren nach Anspruch 1 oder 2, wobei die Schicht Fasern, die in Schritt (a) verwendet werden soll, auf einem Förderband (3), das von einer Kardiereinheit kommt, geführt wird.

4. Verfahren nach irgendeinem der vorhergehenden Ansprüche 1-3, wobei die Düsen (4) der Wasserstrahlen, die in Schritt (a) verwendet werden, einen Durchmesser (φ) von etwa 0,08 mm aufweisen, auf einem ersten rechteckigen Strahlstreifen (5) montiert sind, der über dem Förderband (3) positioniert ist, und eine von der anderen durch eine Entfernung (Abstand, d) gleich etwa 0,8 mm beanstandet ist.

5. Verfahren nach irgendeinem der vorhergehenden Ansprüche 1-4, wobei die Düsen (8), die in Schritt (b) verwendet werden sollen, einen Durchmesser aufweisen, der zwischen 0,8 mm und 0,12 mm (φ)liegt, auf einem zweiten Strahlstreifen (7) montiert sind und eine von der anderen durch einen Abstand (P), der zwischen 1 mm und 5 mm liegt, beanstandet ist, wobei jede der Düsen (8) auch möglicherweise einer oder mehreren Düsen (8) in jedem Abstand (P) benachbart sind.

6. Verfahren nach Anspruch 5, wobei die Düsen (8) in jedem Strahlstreifen (7) in mehreren horizontalen Reihen in einer Entfernung (d) voneinander positioniert sind, wobei jede Düse (8) jeder Reihe einer oder mehreren Düsen (8) in einer Entfernung (f) voneinander benachbart ist, um Gruppen von Düsen (8) zu bilden, wobei jede Gruppe von der anderen durch einen Abstand (P) entfernt ist, der von 1 mm bis 5 mm variiert.

7. Verfahren nach irgendeinem der vorhergehenden Ansprüche, wobei die erste Mehrzahl von Längslinien (1), die eine höhere Faserdichte aufweist, am Ende des Schritts (b) erhalten wird und die zweite Mehrzahl von Längslinien (2), die eine geringere Faserdichte aufweist, am Ende von Schritt (a) erhalten wird.

8. Verfahren nach Anspruch 7, wobei die erste Mehrzahl von Längslinien (1) die eine höhere Faserdichte aufweist und am Ende von Schritt b) erhalten wird, eine geringere Dicke im Vergleich mit der Dicke der zweiten Mehrzahl von Längslinien (2) aufweist, die am Ende von Schritt (a) erhalten wird.

9. Damenbinde mit Flügeln für physiologische Flüssigkeiten, umfassend als Oberblattschicht (10) eine Schicht von absorptionsfähigem Vliesstoffmaterial aus Naturfasern, die durch eine Matte gebildet ist, wobei die Oberblattschicht eine Dichte aufweist, die in der Querrichtung variiert, um eine reduzierte Querkapillarität zu erhalten
das Material mit einer ersten Mehrzahl von Längslinien (1) in Form von Vertiefungen versehen wird, die mit einer zweiten Mehrzahl von Längslinien (2) interkaliert sind, wobei jede der Längslinien (2) mit jeder der Längslinien (1) abwechselt,
**dadurch gekennzeichnet, dass**
die Matte, die das Oberblatt bildet, wasserstrahlverschlungen ist und dass
die Längsvertiefungslinien (1) der ersten Mehrzahl von Längslinien eine höhere Faserdichte im Vergleich mit den Längslinien (2) der zweiten Mehrzahl von Linien aufweisen,
die erste Mehrzahl von Längsvertiefungslinien (1) höherer Faserdichte eine geringere Dicke im Vergleich mit der Dicke der zweiten Mehrzahl von Längslinien (2) aufweist,
wobei das Oberblatt auch die Flügel bildet.

10. Damenbinde nach Anspruch 9, wobei der Dickenunterschied (g/cm³) zwischen der ersten Mehrzahl von Längslinien (1) und der zweiten Mehrzahl von Linien (2) mindestens 10 %, bevorzugt etwa 15-20 % beträgt.

11. Damenbinden nach irgendeinem der vorhergehenden Ansprüche 9-10, wobei die Naturfasern des absorptionsfähigen Materials des Oberblatts (10) aus Baumwolle und/oder irgendeiner anderen Naturfaser und ihren Mischungen mit Viskose, Polymilchsäure (PLA) oder ähnlichen Fasern bestehen.

12. Damenbinde nach Anspruch 9 oder 10 oder 11, wobei der Micronaire-Wert der Baumwollfasern des absorptionsfähigen Materials, das für die Bildung des Oberblatts (10) verwendet wird, zwischen 2 und 5 liegt oder eine Mischung von Fasern mit verschiedenen Micronaire-Werten ist.

13. Damenbinde nach irgendeinem der vorhergehenden Ansprüche 9-12, **dadurch gekennzeichnet, dass** sie Flügel aufweist, die in der Querrichtung entgegengesetzt sind.

14. Oberblatt (10) für Damenbinden für physiologische Flüssigkeiten wie in einem der vorhergehenden Ansprüche 9-13 definiert.

## Revendications

1. Procédé de préparation d'une couche supérieure (10) dans un matériau non tissé absorbant de fibres naturelles pour une serviette hygiénique avec des rabats pour des liquides physiologiques,
ladite couche supérieure (10) ayant une densité qui varie dans le sens transversal et est dotée d'une première pluralité de lignes longitudinales (1) sous la forme de rainures ayant une densité de fibres supérieure et une épaisseur inférieure par rapport à celles d'une seconde pluralité de lignes longitudinales (2), chacune étant alternée avec chaque ligne longitudinale (1),
ledit procédé comprenant
(a) un premier hydro-enchevêtrement d'une couche de fibres avec des pressions inférieures ou égales à 60 bars, avec des jets d'eau adjacents les uns aux autres, afin d'obtenir une légère cohésion entre les fibres due à un tampon légèrement hydro-enchevêtré,
(b) un second hydro-enchevêtrement du tampon légèrement hydro-enchevêtré obtenu à l'étape (a), en utilisant des pressions supérieures ou égales à 100 bars et/ou avec des jets d'eau d'un débit plus élevé, lesdits jets éloignés les uns des autres avec un espacement d'au moins 0,5 mm.

2. Procédé selon la revendication 1, dans lequel la couche de fibres à utiliser dans l'étape (a) est pré-aiguilletée, aiguilletée sous la forme d'un tampon ou constituée par une pluralité de bandes pliées ou cardées.

3. Procédé selon la revendication 1 ou 2, dans lequel la couche de fibres à être utilisée dans l'étape (a) est alimentée par une courroie transporteuse (3) provenant d'une unité de cardage.

4. Procédé selon l'une quelconque des revendications 1 - 3 précédentes, dans lequel les buses (4) pour les jets d'eau utilisés dans l'étape (a) ont un diamètre (Φ) d'environ 0,08 mm, sont montées sur une première bande de jets (5) rectangulaire placée au-dessus de la courroie transporteuse (3) et sont éloignées les unes des autres par une distance (espacement, d) équivalent à environ 0,8 mm.

5. Procédé selon l'une quelconque des revendications 1-4 précédentes, dans lequel les buses (8) à utiliser dans l'étape (b) ont un diamètre compris entre 0,8 mm et 0,12 mm (Φ), sont montées sur une seconde bande de jets (7) et sont éloignées les unes des autres par un espacement (P) compris entre 1 mm et 5 mm, chacune desdites buses (8) étant également éventuellement adjacentes à une ou plusieurs buses (8) au niveau de chaque espacement (P).

6. Procédé selon la revendication 5, dans lequel les buses (8) dans la bande de jets (7) sont placées dans plusieurs colonnes horizontales à une distance (d) les unes des autres, chaque buse (8) de chaque colonne étant adjacente à une ou plusieurs buses (8) à une distance (f) les unes des autres de sorte à former des groupes de buses (8), chaque groupe étant distant de l'eau par un espacement (P) qui varie de 1 mm à 5 mm.

7. Procédé selon l'une quelconque des revendications précédentes, dans lequel la première pluralité de lignes longitudinales (1) ayant une densité de fibre supérieure est obtenue à la fin de l'étape (b) et la seconde pluralité de lignes longitudinales (2) ayant une densité de fibres inférieure est obtenue à la fin de l'étape (a).

8. Procédé selon la revendication 7, dans lequel la première pluralité de lignes longitudinales (1) ayant une densité de fibre supérieure obtenue à la fin de l'étape b) a une épaisseur inférieure par rapport à l'épaisseur de la seconde pluralité de lignes longitudinales (2) obtenue à la fin de l'étape (a).

9. Serviette hygiénique avec des rabats pour des liquides physiologiques comprenant comme couche supérieure (10), une couche de matériau non tissé absorbant de fibres naturelles constituée par un tampon, ladite couche supérieure ayant une densité qui varie dans le sens transversal de sorte à obtenir une capillarité transversale réduite
ledit matériau est doté d'une première pluralité de lignes longitudinales (1), sous la forme de rainures, qui sont intercalées avec une seconde pluralité de lignes longitudinales (2), chacun desdites lignes longitudinales (2) étant alternée avec chaque ligne longitudinale (1),
**caractérisée en ce que**
ledit tampon constituant la couche supérieure est un tampon hydro-enchevêtré,
et **en ce que**
les lignes de rainure longitudinales (1) de ladite première pluralité de lignes longitudinales ont une densité de fibres supérieure par rapport à celle desdites lignes longitudinales (2) de ladite seconde pluralité de lignes,
ladite première pluralité de lignes de rainure longitudinales (1) de densité de fibres supérieure a une épaisseur inférieure par rapport à l'épaisseur de la seconde pluralité de lignes longitudinales (2),
ladite couche supérieure formant également lesdits rabats.

10. Serviette hygiénique selon la revendication 9, dans laquelle la différence en terme de densité (g/cm³) entre ladite première pluralité de lignes longitudinales (1) et ladite seconde pluralité de lignes (2) est d'au moins 10 %, de préférence d'environ 15 - 20%.

11. Serviette hygiénique selon l'une quelconque des revendications 9 - 10, dans laquelle les fibres naturelles du matériau absorbant de la couche supérieure (10) sont du coton et/ou n'importe quelle autre fibre naturelle et leurs mélange avec de la viscose, de l'acide polylactique (PLA) ou d'autres fibres similaires.

12. Serviette hygiénique selon la revendication 9 ou 10 ou 11, dans laquelle le micronaire des fibres de coton du matériau absorbant utilisé pour la formation de la couche supérieure (10) est compris entre 2 et 5, ou est un mélange de fibres de différent micronaire.

13. Serviette hygiénique selon l'une quelconque des revendications 9 - 12, **caractérisée en ce qu'**elle comprend des rabats qui sont opposée audit sens transversal.

14. Couche supérieure (10) pour serviettes hygiéniques pour des liquides physiologiques selon l'une quelconque des revendications 9-13.
